# EUROPÄISCHE PATENTSCHRIFT

(11) **EP 3 948 401 B1**
(45) Veröffentlichungstag und Bekanntmachung des Hinweises auf die Patenterteilung: **09.10.2024**
(21) Anmeldenummer: 20785897.8
(22) Anmeldetag: 26.05.2020
(51) Int. Cl.: G02B 27/01, A61B 90/25, A61B 90/20, A61B 34/20, A61B 90/00

(54) **VIDEOBRILLE ZUR VERWENDUNG MIT EINEM STEREOMIKROSKOP FÜR MIKROCHIRURGISCHE EINGRIFFE AM PATIENTEN**
VIDEO GLASSES FOR USE WITH A STEREO MICROSCOPE FOR MICROSURGICAL PROCEDURES ON A PATIENT
LUNETTES VIDÉO CONÇUES POUR ÊTRE UTILISÉES AVEC UN MICROSCOPE STÉRÉOSCOPIQUE POUR EFFECTUER DES INTERVENTIONS MICROCHIRURGICALES SUR DES PATIENTS

(30) Priorität: 29.05.2019 AT 2042019
(43) Veröffentlichungstag der Anmeldung: 09.02.2022
(73) Patentinhaber: BHS Technologies GmbH, 6020 Innsbruck (AT)
(72) Erfinder: HÜTTER, Markus Friedrich, 6170 Zirl (AT)
(74) Vertreter: Prüfer & Partner mbB Patentanwälte · Rechtsanwälte
(86) Internationale Anmeldenummer: PCT/AT2020/000011
(87) Internationale Veröffentlichungsnummer: WO 2020/237267

(56) Entgegenhaltungen:
- WO-A1-2010/126441
- WO-A1-2018/170522
- US-A1- 2002 120 979
- US-A1- 2005 063 047
- US-A1- 2007 114 252
- US-A1- 2010 299 814
- US-A1- 2016 225 192

## Beschreibung

### TECHNISCHES FELD

Die vorliegende Erfindung betrifft eine Videobrille für die Verwendung mit einem Stereomikroskop für mikrochirurgische Eingriffe am Patienten.

### HINTERGRUND DER ERFINDUNG

Auf dem Gebiet der Chirurgie an kleinsten Strukturen, beispielsweise in der Neurochirurgie oder Eingriffe an den Gehörknöchelchen im Mittelohr, sowie in der Implantationschirurgie ist die Mikroskop-unterstützte chirurgische Operation bekannt. Dabei wird die zu behandelnde Stelle, im folgenden Eingriffsbereich bezeichnet, am Patienten an der der mikrochirurgische Eingriff zu erfolgen hat mithilfe eines Mikroskops vergrößert dem Chirurgen bevorzugt mittels eine Videobrille dargestellt.

Aus der anhängigen Patentanmeldung ATS 19845 oder der WO2018/170522 der Erfinder ist eine Videobrille bekannt zur Verwendung mit einem Stereomikroskop. Es hat sich herausgestellt, dass bei dieser Videobrille durch das zusätzliche Schaffen der Möglichkeit die Bildwiedergabeeinheit manuell aus und in das Blickfeld des die Videobrille tragenden Chirurgen zu Verschwenken ohne nennenswerten Widerstand und gleichzeitig die Anforderung im nach oben herausgeschwenkten Zustand der Bildwiedergabeeinheit diese stabil zu halten nicht miteinander vereinbar sind. Beziehungsweise nur durch eine zusätzliche aufwendige Steuerungsschaltung mit Sensoren und Antrieben zu realisieren ist.

Es besteht daher der Bedarf eine Videobrille mit leicht und ohne nennenswerten Widerstand manuell jederzeit verschwenkbare Bildwiedergabeeinheit bereitzustellen, bei gleichzeitig stabiler Lagehaltung der Bildwiedergabeeinheit in zumindest einer Haltestellung auch bei schnellen Bewegungen des die Videobrille tragenden Chirurgen und damit verbunden hohen auftretenden Beschleunigungs- und Fliehkräften.

### ZUSAMMENFASSUNG DER ERFINDUNG

Diese Aufgabe wird mit der erfindungsgemäßen Videobrille durch die Merkmale des Anspruchs 1 erreicht. Vorteilhafte Weiterbindungen sind in den abhängigen Ansprüchen 1 bis 14 angegeben. Anspruch 15 gibt ein erfindungsgemäßes Stereomikroskop an.

Eine erfindungsgemäße Videobrille zur Verwendung bei mikrochirurgischen Eingriffen umfasset eine Stützvorrichtung eingerichtet um die Videobrille am Kopf eines Benutzers zu befestigen, eine Bildwiedergabeeinheit zur Ausgabe von Bildern,
ein Gelenk mit einer Drehachse das die Haltevorrichtung mit der Bildwiedergabeeinheit zueinander zwischen einer Betriebsstellung in der die Bildwiedergabeeinheit vor den Augen des Benutzers angeordnet ist und die Bilder dem Benutzer angezeigt werden können und einer Ruhestellung in der die Bildwiedergabeeinheit nach oben aus dem Blickfeld des Benutzers angeordnet ist um die Drehachse drehbar verbindet und
eine Verriegelungsvorrichtung umfassend eine Einrastvorrichtung und eine Raste derart angeordnet um bei Drehung in eine Drehrichtung um die Drehachse die Einrastvorrichtung in einer Haltestellung in der Raste einrasten zu lassen und bei weiterer Drehung in die Drehrichtung die Einrastvorrichtung wieder aus der Raste löst und wobei eine Drehung in die entgegengesetzte Drehrichtung über die Haltestellung hinaus nicht möglich ist, solange die Einrastvorrichtung in der Raste eingerastet ist.

In einer Ausführungsform entspricht die Haltestellung der Ruhestellung und/oder die Verriegelungseinrichtung weist eine weitere Haltestellung zwischen der Ruhestellung und der Betriebsstellung auf. In einer vorteilhaften Ausführungsform ist der Drehwinkel von der Haltestellung bis zum Lösen der Einrastvorrichtung aus der Raste zumindest 10°. Der Drehwinkel kann in einer weiteren vorteilhaften Ausführungsform nicht mehr als 15° betragen oder im Bereich von 10° bis 15°.

Die Einrastvorrichtung kann als elastisch verformbare Zunge ausgebildet sein und in einer weiteren vorteilhaften Ausführungsform einstückig mit der Stützvorrichtung aus Kunststoff gefertigt sein. In einer alternativen Ausführungsform kann die Einrastvorrichtung ein mittels Feder vorgespannten und drehbar gelagerten Stift umfassen.

In einer weiteren bevorzugten Ausführungsform ist die Verriegelungsvorrichtung derart ausgebildet, um nur in der Drehrichtung von der Betriebsstellung in die Ruhestellung im eingerasteten Zustand eine Drehung in die entgegengesetzte Drehrichtung über die Haltestellung hinaus zu unterbinden. Das kann in einer Ausführungsform dadurch erreicht werden, dass die Einrastvorrichtung ein an einer Seite abgerundeten Stift umfasst und die Raste derart ausgebildet ist, um den abgerundeten Stift aus der Raste gleiten zu lassen bei Drehung in entgegengesetzter Drehrichtung.

Die Erfindungsgemäße Videobrille kann eine Raste aufweisen, die als Einkerbung ausgebildet ist. Diese Einkerbung kann an der Spitze einer Nase angebracht sein. In einer bevorzugten Weiterbildung der Videobrille ist das Gelenk ein Parallellogramm-Gelenk. Die erfindungsgemäße Videobrille kann zudem einen Orientierungssensor aufweisen. Das erfindungsgemäße Stereomikroskop umfassend eine erfindungsgemäße Videobrille.

### KURZE BESCHREIBUNG DER ZEICHUNGEN

Abbildung 1 zeigt ein mikrochirurgisches Stereomikroskop in einer Ausführungsform zur Verwendung der Videobrille gemäß der Erfindung.
Abbildung 2 zeigt eine aus dem Stand-der-Technik bekannte Videobrille.
Abbildung 3 zeigt eine Videobrille in einer für das erfindungsgemäße Stereomikroskop bevorzugten Ausführungsform.
Abbildungen 4A-D zeigt eine bevorzugte Ausführungsform der erfindungsgemäßen Verriegelungsvorrichtung der Videobrille in unterschiedlichen Stellungen.
Abbildung 5 zeigt eine 3D-Ansicht der erfindungsgemäßen Videobrille in einer Ausführungsform..

### BESCHREIBUNG DER BEVORZUGTEN AUSFÜHRUNGSFORMEN ANHAND DER ABBILDUNGEN

Im Folgenden werden bevorzugte Ausführungsformen der erfindungsgemäßen Videobrille 105 zur Verwendung mit einem Stereomikroskop 100 bei mikrochirurgischen Eingriffen anhand der beigefügten Abbildungen beschrieben.

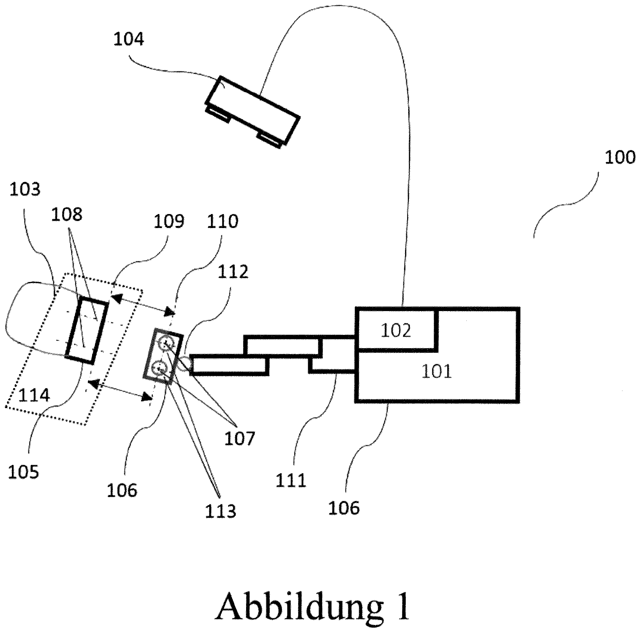

Abbildung 1 zeigt schematisch den Aufbau eines Stereomikroskops 100 der Erfinder in der anhängigen Patentanmeldung mit der Anmeldenummer PCT2019AT000005 in der Draufsicht zur Verwendung bei mikrochirurgischen Eingriffen mit welchem die erfindungsgemäße Videobrille bevorzugt verwendet werden kann. Das Stereomikroskop 100 umfasst ein Stativ 106 das schwenkbar über ein Gelenk 112 mit einem Roboterarm 111 verbindbar ist. Der Roboterarm 111 ist über eine Haltevorrichtung 106 stabil auf dem Boden des Operationssaales fixierbar um Schwingungen weitestgehend zu vermeiden. Mit dem Stativ 106 sind zwei optische Bilderfassungseinheiten 113 derart verbindbar um ein stereoskopisches Bild des während der Benutzung abzubildenden Eingriffsbereichs 117 am Patienten erfassen zu können und durch die zwei optischen Achsen 107 der Bilderfassungseinheiten 113 eine Bildaufnahmeebene 110 definierend. Die Bilderfassungseinheiten 113 sind eingerichtet um eine bis zu 100-fache optische Vergrößerung zu ermöglichen. In einer vorteilhaften Ausführungsform können die Bilderfassungseinheiten 113 zusätzlich dazu eingerichtet sein eine optische Verkleinerung zu ermöglichen. Das ist insbesondere für den Benutzer 103 von Vorteil, da er sich einen Überblick über den Eingriffsbereich 117 und darüber hinaus verschaffen kann. Auch hilft es dem Benutzer 103 sich zu orientieren, wenn er sich mit den chirurgischen Werkzeugen in den Händen dem Eingriffsbereich 117 nähert. Die von den beiden Bilderfassungseinheiten 113 erfassten Bilder werden mit einer Videobrille 105, umfassend eine optische Bildwiedergabeeinheit 115 mit einer Anzeige zur Wiedergabe eines Bildes, einem die Videobrille 105 tragenden Benutzer 103 übermittelt und angezeigt. Zur stereoskopisch korrekten, und für die natürliche Augen-Hand-Koordination erforderliche Ausrichtung der dem Benutzer angezeigten Bilder, weist das Stereomikroskop eine Erfassungseinrichtung 104 auf, welche die räumliche Orientierung der Videobrille 105 erfasst.

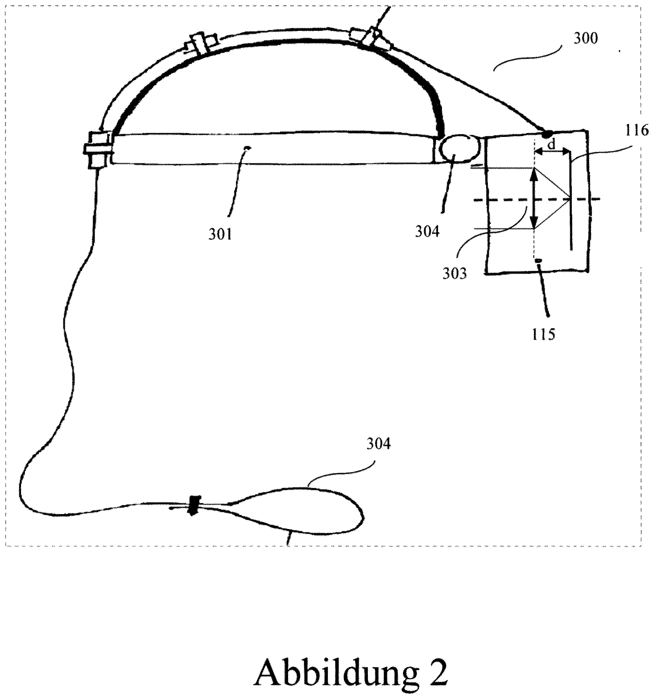

Abbildung 2 zeigt eine Videobrille 300 mit Stützvorrichtung 301 zum Halten der Videobrille 300 am Kopf des Benutzers 103 wie sie aus der Patentanmeldung der Erfinder AT519845 bekannt ist. Die Bildwiedergabeeinheit 115 ist über Gelenk 304 mit einer Drehachse 302 drehbar gegenüber der Stützvorrichtung 301 verbunden. Die Videobrille 300 umfasst zusätzlich einen Seilzug 304 der mit einem Ende eine manuell Bedienbare Schlaufe bildet und mit dem anderen Ende an der Bildwiedergabeeinheit 115 in der Weise befestigt, dass die Bildwiedergabeeinheit 115 mit dem Gelenk 304 um die Drehachse 302 nach oben derart schwenkbar ist, um aus dem Blickfeld des die Videobrille 300 tragenden Benutzers 103 in die Ruhestellung herausgeführt werden kann. In der Ausführungsform in Abbildung 2 geschieht das dadurch, dass durch ziehen am Seilzug 304, die Seillänge verkürzt wird. Damit wird die Bildwiedergabeeinheit 115 die zunächst vor dem Auge des die Videobrille 300 tragenden Benutzers 103 angeordnet ist und sich in der Betriebsstellung befindet, entgegen der Gewichtskraft F über das Gelenk 304 um die Drehachse 302 nach oben über die Augen und damit aus dem Blickfeld des Benutzers 103 in die Ruhestellung geschwenkt. Wird umgekehrt die Seillänge verlängert, so schwenkt die Bildwiedergabeeinheit 115 von der Ruhestellung infolge der Gewichtskraft F in die Betriebsstellung zurück. Die Erfindungsgemäße Videobrille 300 ist dabei keineswegs auf diese Ausführungsform eingeschränkt, insbesondere sind andere Antriebe und auch manuelle Interventionen möglich um die Bildwiedergabeeinheit 115 aus dem Blickfeld des Benutzers in die Ruhestellung (nicht dargestellt) herauszuführen und umgekehrt von der Ruhestellung in die Betriebsstellung zu bringen. Die Bildwiedergabeeinheit 115 umfasst Bildanzeigevorrichtungen 116 und je Auge des Benutzers eine Sammellinse 303. Die Bildanzeigevorrichtung 116 der Bildwiedergabeeinheit 115 sind in einer Ebene, die Bildebene 109 bildend, angeordnet und können entweder als ein einziges Display, wobei das auf dem Display ausgegebene Bild für je eine Bildwiedergabeeinheit 115 geteilt wird, oder als zwei getrennte Displays ausgebildet sein.

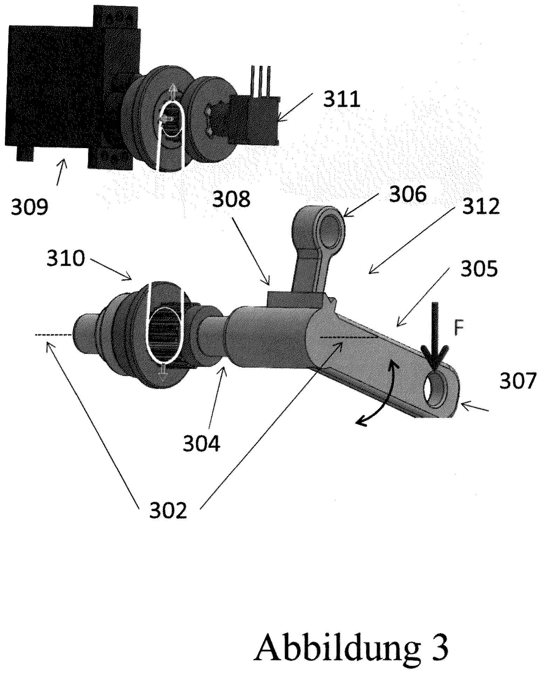

Abbildung 3 zeigt beispielhaft eine Ausführungsform der Verriegelungsvorrichtung 312 der erfindungsgemäßen Videobrille 300. Dabei ist Gelenk 304 mit der Drehachse 302 an einer Seite über den Hebelarm 305 mit der Bildwiedergabeeinheit 115 über eine mechanische Verbindung 307 fixiert. Auf der anderen Seite ist das Gelenk 304 an der nicht dargestellten Stützvorrichtung 301 fixiert sodass eine Drehung des Gelenks 304 um die Drehachse 302 die Bildwiedergabeeinheit 115 relativ zur Stützvorrichtung 301 verschwenkt. Ist wie in der Abbildung dargestellt Gelenk 304 ein Drehgelenk so wird die Bildwiedergabeeinheit 115 um die Gelenkachse 302 dabei gedreht. Wird das Gelenk 304 in einer Ausführungsform als ParallelogrammGelenk (nicht gezeigt) ausgebildet, so bewegt sich die Bildwiedergabeeinheit 115 auf einer Kreisbahn ohne sich dabei selbst um die Drehachse 302 zu drehen. Dadurch kann die Bildwiedergabeeinheit 115 in der Ruhestellung näher am Kopf des Benutzers zu liegen kommen, bildet einen kürzeren Hebel und lässt auf den Kopf des Benutzers in vorteilhafter weise ein geringeres Drehmoment wirken. Das erhöht den Tragekomfort der Videobrille 300 in der Ruheposition.

In einer bevorzugten Ausführungsform umfasst die erfindungsgemäße Videobrille 300 einen elektrischen Antrieb 309 der über eine Rutschkupplung 310 eine drehbare Verbindung mit dem Gelenk 304 herstellt. Die Rutschkupplung 310 hat dabei die Aufgabe den elektrischen Antrieb 309, wenn sich die Bildwiedergabeeinheit 115 in der Ruhestellung oder Betriebsstellung befindet, nicht anzuhalten um hohe Ströme durch den elektrischen Antrieb 309 zu vermeiden. Zusätzlich hilft die Rutschkupplung 310 die anzuwendende Kraft für die manuelle Verschwenkung der Bildwiedergabeeinheit 115 zu verringern. Beispielsweise ist die vom Benutzer 103 aufzuwendende Kraft um die Bildwiedergabeeinheit 115 von der Betriebsstellung nach oben in die Ruhestellung zu bringen nur geringfügig größer als die Gewichtskraft F. Zudem verbessert die Rutschkupplung 310 die Sicherheit, falls die Bildwiedergabeeinheit 115 in einer Zwischenstellung zwischen Betriebsstellung und Antriebstellung blockiert wird, um wie zuvor einerseits zu hohe Ströme durch den elektrischen Antrieb 309 zu vermeiden und andererseits den Benutzer 103 vor dem Einklemmen zu bewahren. Die drehbare Verbindung kann entweder durch direkte Verbindung von Gelenk 304 und elektrischen Antrieb 309 erfolgen, oder durch ein zwischengeschaltetes Untersetzungsgetriebe bestehend aus ineinandergreifenden Zahnrädern oder Riemen oder eine Kombination aus Zahnrädern und Riemen. Der elektrische Antrieb 306 kann in einer Ausführungsform durch einen Servomotor ausgebildet sein.

Der elektrische Antrieb 309 ist in einer vorteilhaften Ausführungsform der Erfindung an der Stützvorrichtung 301 fixiert und treibt den Teil vom Gelenk 304 an, der mit der Bildwiedergabeeinheit 115 fixiert ist. Diese Ausführungsform hat den Vorteil, dass das Gewicht der Bildwiedergabeeinheit 115 verringert werden kann und trägt somit bei das die Bildwiedergabeeinheit 115 mit einem geringeren Kraftaufwand sowohl mit dem elektrischen Antrieb 309 als auch manuell verschwenkt werden kann. Der elektrische Antrieb 309 kann aber genauso gut an der Bildwiedergabeeinheit 115 fixiert sein, wobei hier derjenige Teil des Gelenks 304 angetrieben wird, der mit der Stützvorrichtung 301 verbunden ist. Zur Lagekontrolle der Bildwiedergabeeinheit 115 gegenüber der Stützvorrichtung 301 kann ein Sensor 311 auf der Drehachse des elektrischen Antriebs 309 oder der Drehachse 302 des Gelenks 304 angebracht werden. In einer Ausführungsform kann der Sensor 311 ein Drehratensensor oder ein Drehwiderstand sein.

Die Verriegelungsvorrichtung 312 umfasst eine Einrastvorrichtung 306 und eine Raste 308, die derart angeordnet ist um bei Drehung in einer Drehrichtung um die Drehachse 302 des Gelenks 304 die Einrastvorrichtung 306 in der Haltestellung in der Raste 308 einrasten zu lassen sodass dadurch eine Drehung in die entgegengesetzte Drehrichtung über die Haltestellung hinaus unterbunden wird und bei weiterer Drehung in der Drehrichtung die Einrastvorrichtung 306 sich wieder aus der Raste 308 löst sodass eine Drehung in die entgegengesetzte Drehrichtung über die Haltestellung hinaus wieder möglich wird. Die Einrastvorrichtung 306 kann dabei entweder an der Stützvorrichtung 301 fixiert sein und die Raste 308 an der Bildwiedergabeeinheit 115 oder umgekehrt die Einrastvorrichtung 306 an der Bildwiedergabeeinheit 115 und die Raste 308 an der Stützvorrichtung 301 fixiert. an der Die Haltestellung ist dabei entweder eine Zwischenstellung zwischen der Betriebsstellung und der Ruhestellung oder in einer Ausführungsform die Ruhestellung selbst. In einer weiteren Ausführungsform kann die Verriegelungseinrichtung 312 zumindest eine weitere Haltestellung zwischen der Ruhestellung und der Betriebsstellung aufweisen.

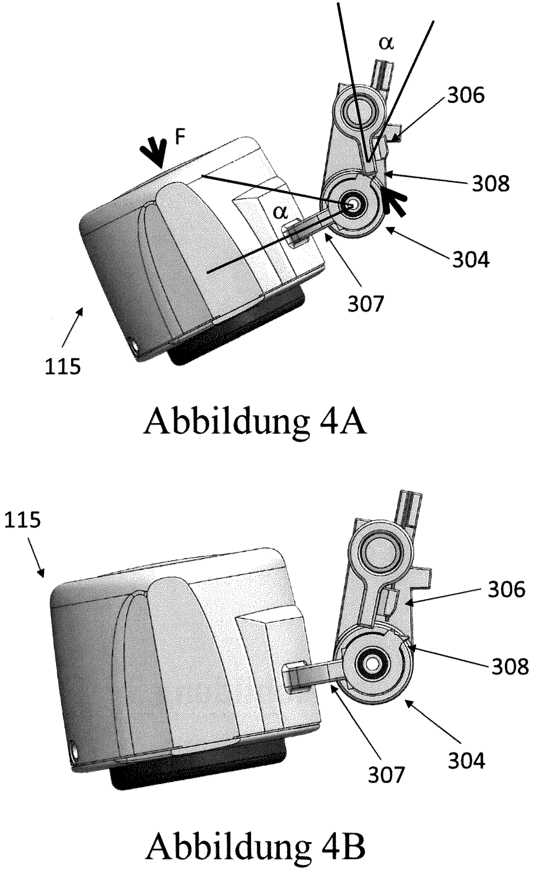

Im Folgenden wird die Erfindungsgemäße Videobrille 300 anhand Abbildungen 4A-D erläutert. Die Abbildungen 4A-D zeigen die Verriegelungsvorrichtung 312 in unterschiedlichen Stellungen. Abbildung 4A zeigt die Verriegelungsvorrichtung 312 in der Haltestellung wie sie nach der Drehung von der Betriebsstellung in die Ruhestellung, in der Abbildung entspricht das einer Drehung im Uhrzeigersinn, zu liegen kommt. Die Einrastvorrichtung 306, hier in einer Ausführungsform gezeigt als vorgespannter und drehbarer Stift, ist in der Raste 308 eingerastet. Die Einrastvorrichtung 306 kann in einer Ausführungsform eine elastische Zunge sein, bevorzugt einstückig mit der Stützvorrichtung 301. Die Elastizität übernimmt dabei die Federwirkung. Raste 308 ist in dieser Ausführungsform als Nase ausgebildet, die von der Drehachse 304 des Gelenks 302 radial nach außen absteht und an der Spitze eine Einkerbung aufweist. In der Haltestellung ist die Einrastvorrichtung 306 in der Einkerbung, wie gezeigt, eingerastet. Die Gewichtskraft F der Bildwiedergabeeinheit wirkt entgegen dieser Drehrichtung. Die Raste 308 stützt sich gegen die Einrastvorrichtung 306 ab (angedeutet durch den Pfeil) und unterbindet so eine Drehung der Bildwiedergabeeinheit 115 zurück in Richtung Betriebsstellung über die Haltestellung hinaus. Das entspricht in der Abbildung einer Drehung um die Drehachse 304 gegen den Uhrzeigersinn.

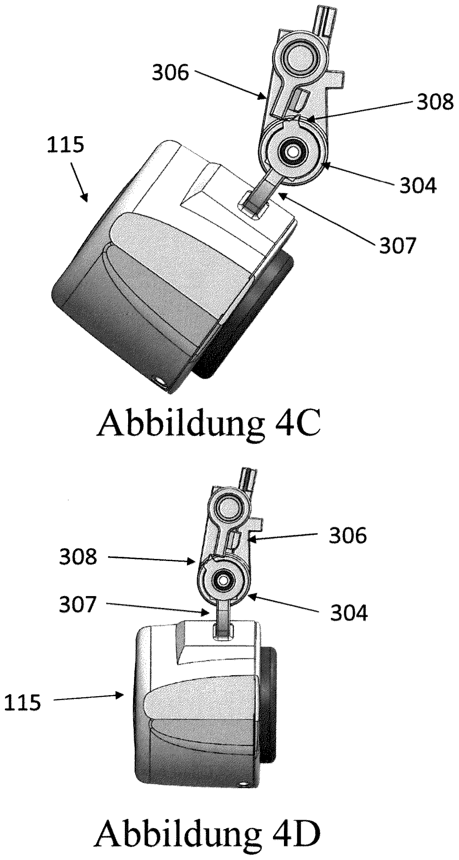

Abbildung 4B zeigt die Verriegelungsvorrichtung 312 wenn sie in Drehrichtung weitergedreht wird, entsprechend in der Abbildung einer Drehung im Uhrzeigersinn. Die Einrastvorrichtung 306 verbleibt zunächst noch in der Raste 308. Erst wenn die Verdrehung über einen Winkel α hinaus weitergedreht ist, löst sich die Einrastvorrichtung 306 wieder aus der Raste 308. In einer bevorzugten Ausführungsform ist der Winkel α etwa 10°, 15° oder im Bereich von 10° bis 15°. Wird nun wie in Abbildung 4C dargestellt gegen den Uhrzeigersinn gedreht, so wird die Einrastvorrichtung 306 von der umgekehrten Seite in die Raste 308 eingreifen und einrasten. Dementsprechend stützt sich die Raste 308 gegen die Einrastvorrichtung 306 nur wieder bei einer Drehung in die entgegengesetzte Drehrichtung ab. Das ist in der Abbildung eine Drehung im Uhrzeigersinn. Wird gegen den Uhrzeigersinn weitergedreht, so löst sich abermals die Einrastvorrichtung 306 aus der Raste 308 und eine weitere Drehung um die Drehachse 304 des Gelenks 302 kann ohne Einschränkungen erfolgen bis die Bildwiedergabeeinheit 115 wieder in der Betriebsstellung zu liegen kommt. Diese Stellung ist in Abbildung 4D gezeigt. Wird die Bildwiedergabeeinheit 115 wieder Richtung Ruhestellung durch Drehung um die Drehachse 304 verschwenkt, wird die Verriegelungsvorrichtung 312 wieder in die Haltestellung wie in Abbildung 4A gezeigt gebracht.

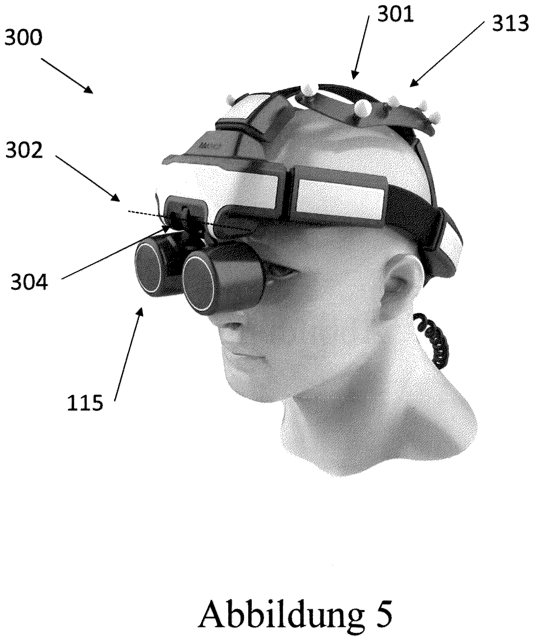

Abbildung 5 zeigt eine Ausführungsform der erfindungsgemäßen Videobrille 300 umfassend eine Stützvorrichtung 301, eine Bildwiedergabeeinheit 115, ein Gelenk 304 mit der Drehachse 302 und eine optische Markierung 313 an vorbestimmten Positionen auf der Videobrille 300 zur Erfassung der räumlichen Orientierung der Videobrille 300 mit der Erfassungseinrichtung 104 aufweist. Die Markierungen können beispielsweise farbig oder im infraroten Bereich reflektierende Punkte, Linien oder andere per Bilderkennung erkennbare Symbole sein. Die Drehachse 302 ist parallel zur Stirn des Benutzers 103 wenn die Videobrille 300 getragen wird. Die Bildwiedergabeeinheit 115 befindet sich in der Betriebsstellung vor den Augen des Benutzers 103 angeordnet. Wird die Bildwiedergabeeinheit 115 mit dem Gelenk 304 über Drehachse 302 nach oben verdreht, so kommt die Bildwiedergabeeinheit über den Augen und außerhalb des Blickfeldes des Benutzers 103 in der Ruhestellung zu liegen. In dieser Lage befindet sich die Videobrille 300 in dieser Ausführungsform zudem in der Halteposition und die Einrastvorrichtung 306 ist in der Raste 308 der Verriegelungsvorrichtung 312 eingerastet und unterbindet eine Drehung Richtung Betriebsstellung über die Haltestellung, die in diesem Fall zugleich die Ruhestellung ist, hinaus. Die Bildwiedergabeeinheit 115 wird dadurch stabil in der Ruheposition gehalten. Um die Bildwiedergabeeinheit 115 wieder in die Betriebsstellung bringen zurück bringen zu können, muss zunächst die Bildwiedergabeeinheit 115 entweder manuell oder mit dem elektrischen Antrieb 309 über die Ruhestellung hinaus durch Drehung des Gelenks 302 um die Gelenkachse 304 weiter nach oben angehoben werden, bis sich die Einrastvorrichtung 306 wieder aus der Raste 308 löst. Erst dann kann die Bildwiedergabeeinheit 115 in umgekehrter Drehrichtung des Gelenks 304 um die Drehachse 302 wieder in die Betriebsstellung gebracht werden.

## Patentansprüche

1. Videobrille (105, 300) zur Verwendung bei mikrochirurgischen Eingriffen umfassend eine Stützvorrichtung (301) eingerichtet um die Videobrille (105, 300) am Kopf eines Benutzers (103) zu befestigen, eine Bildwiedergabeeinheit (115) zur Ausgabe von Bildern, ein Gelenk (304) mit einer Drehachse (302), das die Stützvorrichtung (301) mit der Bildwiedergabeeinheit (115) zueinander zwischen einer Betriebsstellung, in der die Bildwiedergabeeinheit vor den Augen des Benutzers (103) angeordnet ist und die Bilder dem Benutzer (103) angezeigt werden können, und einer Ruhestellung, in der die Bildwiedergabeeinheit (115) nach oben aus dem Blickfeld des Benutzers (103) angeordnet ist, um die Drehachse (302) drehbar verbindet, und eine Verriegelungsvorrichtung (312),
**dadurch gekennzeichnet, dass** die Verriegelungsvorrichtung (312) eine Einrastvorrichtung (306) und eine Raste (308) derart angeordnet, um bei Drehung in eine Drehrichtung um die Drehachse (302) die Einrastvorrichtung (306) in einer Haltestellung in der Raste (308) einrasten zu lassen und bei weiterer Drehung in die Drehrichtung die Einrastvorrichtung (306) wieder aus der Raste (308) zu lösen, umfasst, und wobei eine Drehung in die entgegengesetzte Drehrichtung über die Haltestellung hinaus nicht möglich ist, solange die Einrastvorrichtung (306) in der Raste (308) eingerastet ist.

2. Videobrille (105, 300) nach Anspruch 1, wobei die Haltestellung der Ruhestellung entspricht.

3. Videobrille (105, 300) nach Anspruch 1 oder 2, wobei die Verriegelungseinrichtung (312) zumindest eine weitere Haltestellung zwischen der Ruhestellung und der Betriebsstellung aufweist.

4. Videobrille (105, 300) nach einem der vorhergehenden Ansprüche, wobei der Drehwinkel von der Haltestellung bis zum Lösen der Einrastvorrichtung (306) aus der Raste (308) zumindest 10° beträgt.

5. Videobrille (105, 300) nach einem der vorhergehenden Ansprüche, wobei der Drehwinkel von der Haltestellung bis zum Lösen der Einrastvorrichtung (306) aus der Raste (308) nicht mehr als 15° beträgt.

6. Videobrille (105, 300) nach einem der vorhergehenden Ansprüche, wobei die Einrastvorrichtung (306) als elastisch verformbare Zunge ausgebildet ist.

7. Videobrille (105, 300) nach Anspruch 6, wobei die elastische Zunge einstückig mit der Stützvorrichtung (301) aus Kunststoff gefertigt ist.

8. Videobrille (105, 300) nach einem der Ansprüche 1 bis 5, wobei die Einrastvorrichtung (306) einen mittels Feder vorgespannten und drehbar gelagerten Stift umfasst.

9. Videobrille (105, 300) nach einem der vorhergehenden Ansprüche, wobei die Verriegelungsvorrichtung (312) derart ausgebildet ist, um nur in der Drehrichtung von der Betriebsstellung in die Ruhestellung im eingerasteten Zustand eine Drehung in die entgegengesetzte Drehrichtung über die Haltestellung hinaus zu unterbinden.

10. Videobrille (105, 300) nach Anspruch 9, wobei die Einrastvorrichtung (306) ein an einer Seite abgerundeten Stift umfasst und die Raste (308) derart ausgebildet ist, um den abgerundeten Stift aus der Raste (308) gleiten zu lassen bei Drehung in entgegengesetzter Drehrichtung.

11. Videobrille (105, 300) nach einem der vorhergehenden Ansprüche, wobei die Raste (308) eine Einkerbung umfasst.

12. Videobrille (105, 300) nach Anspruch 11, wobei die Einkerbung an der Spitze einer Nase angebracht ist.

13. Videobrille (105, 300) nach einem der vorhergehenden Ansprüche, wobei das Gelenk (304) ein Parallellogramm-Gelenk umfasst.

14. Videobrille (105, 300) nach einem der vorhergehenden Ansprüche, wobei die Videobrille (105) einen mit der Videobrille (105) verbindbaren Orientierungssensor umfasst.

15. Stereomikroskop (100) umfassend eine Videobrille (105, 300) nach einem der vorhergehenden Ansprüche.

## Claims

1. Video glasses (105, 300) for use in microsurgical procedures, comprising:
a support device (301) configured to fasten the video glasses (105, 300) to a head of a user (103),
an image display unit (115) for outputting images,
a joint (304) with a rotational axis (302), which connects the support device (301) with the image display unit (115) rotationally about the rotational axis (302) between an operating position, in which the image display unit is arranged in front of the eyes of the user (103) and images can be displayed to the user (103), and a rest position, in which the image display unit (114) is arranged upwards out of the visual field of the user (103), and
a locking device (312),
**characterized in that**
the locking device (312) comprising a latching device (306) and a catch (308) arranged to let the latching device (306) upon rotation in a rotational direction about the rotational axis (302) engage the catch (308) in a holding position and to release the latching device (306) from the catch (308) upon further rotation in the rotational direction, and
wherein a rotation in the opposite rotational direction beyond the holding position is impeded as long as the latching device (306) is engaged in the catch (308).

2. Video glasses (105, 300) according to claim 1, wherein the holding position corresponds to the rest position.

3. Video glasses (105, 300) according to claim 1 or 2, wherein the locking device (312) comprises at least a further holding position between the rest position and the operating position.

4. Video glasses (105, 300) according to any one of the preceding claims, wherein the rotational angle from the holding position until release of the latching device (306) from the catch (308) is at least 10°.

5. Video glasses (105, 300) according to any one of the preceding claims, wherein the rotational angle from the holding position until release of the latching device (306) from the catch (308) is not more than 15°.

6. Video glasses (105, 300) according to any one of the preceding claims, wherein the latching device (306) is configured as an elastically deformable tongue.

7. Video glasses (105, 300) according to claim 6, wherein the elastic tongue is integrally made of plastic with the support device (301).

8. Video glasses (105, 300) according to any one of the claims 1 to 5, wherein the latching device (306) comprises a pin biased by a spring and being rotationally supported.

9. Video glasses (105, 300) according to any one of the preceding claims, wherein the locking device (312) is configured to only impede a rotation in the opposite rotational direction beyond the holding position in the rotational direction from the operating position into the rest position in the engaged state.

10. Video glasses (105, 300) according to claim 9, wherein the latching device (306) comprises a pin rounded at one side and the catch (308) is configured to let the rounded pin slide off the catch (308) upon rotation in the opposite rotational direction.

11. Video glasses (105, 300) according to any one of the preceding claims, wherein the catch (308) comprises a notch.

12. Video glasses (105, 300) according to claim 11, wherein the notch is applied at a tip of a nose.

13. Video glasses (105, 300) according to any one of the preceding claims, wherein the joint (304) comprises a parallelogram joint.

14. Video glasses (105) according to any one of the preceding claims, wherein the video glasses (105) comprise an orientation sensor connectable to the video glasses (105).

15. Stereoscopic microscope (100) comprising video glasses (105, 300) according to any one of the preceding claims.

## Revendications

1. Lunettes vidéo (105, 300) destinées à être utilisées en microchirurgie, comprenant un dispositif de support (301) conçu pour fixer les lunettes vidéo (105, 300) à la tête d'un utilisateur (103), une unité de lecture d'image (115) pour la sortie d'images, une articulation (304) avec un axe de rotation (302) reliant le dispositif de support (301) à l'unité de lecture d'image (115) entre une position de fonctionnement dans laquelle l'unité de lecture d'image est placée devant les yeux de l'utilisateur (103) et les images peuvent être affichées à l'utilisateur (103), et une position de repos dans laquelle l'unité de lecture d'image (115) est placée vers le haut à partir du champ de vision de l'utilisateur (103), de manière à pouvoir tourner autour de l'axe de rotation (302), et un dispositif de verrouillage (312),
**caractérisées en ce que** le dispositif de verrouillage (312) comprend un dispositif d'encliquetage (306) et un encliquetage (308) agencé de telle sorte que, lorsqu'il tourne dans un sens de rotation autour de l'axe de rotation (302), le dispositif d'encliquetage (306) s'enclenche dans une position d'arrêt dans l'encliquetage (308) et, lors d'une rotation ultérieure dans le sens de rotation, le dispositif d'encliquetage (306) soit à nouveau libéré de l'encliquetage (308), et dans lesquelles une rotation dans le sens de rotation opposé au-delà de la position d'arrêt n'est pas possible tant que le dispositif d'encliquetage (306) est enclenché dans l'encliquetage (308).

2. Lunettes vidéo (105, 300) selon la revendication 1, dans lesquelles la position de maintien correspond à la position de repos.

3. Lunettes vidéo (105, 300) selon la revendication 1 ou 2, dans lesquelles le dispositif de verrouillage (312) présente au moins une autre position de maintien entre la position de repos et la position de fonctionnement.

4. Lunettes vidéo (105, 300) selon l'une quelconque des revendications précédentes, dans lesquelles l'angle de rotation entre la position de maintien et le détachement du dispositif d'encliquetage (306) de l'encliquetage (308) est d'au moins 10°.

5. Lunettes vidéo (105, 300) selon l'une quelconque des revendications précédentes, dans lesquelles l'angle de rotation entre la position de maintien et le détachement du dispositif d'encliquetage (306) de l'encliquetage (308) n'est pas supérieur à 15°.

6. Lunettes vidéo (105, 300) selon l'une quelconque des revendications précédentes, dans lesquelles le dispositif d'encliquetage (306) est conçu comme une languette élastiquement déformable.

7. Lunettes vidéo (105, 300) selon la revendication 6, dans lesquelles la languette élastique est réalisée d'un seul tenant avec le dispositif de support (301) en matière plastique.

8. Lunettes vidéo (105, 300) selon l'une quelconque des revendications 1 à 5, dans lesquelles le dispositif d'encliquetage (306) comprend une broche précontrainte par ressort et montée rotative.

9. Lunettes vidéo (105, 300) selon l'une quelconque des revendications précédentes, dans lesquelles le dispositif de verrouillage (312) est conçu pour empêcher une rotation dans le sens de rotation opposé au-delà de la position de maintien uniquement dans le sens de rotation de la position de fonctionnement à la position de repos à l'état enclenché.

10. Lunettes vidéo (105, 300) selon la revendication 9, dans lesquelles le dispositif d'encliquetage (306) comprend une broche arrondie sur un côté et l'encliquetage (308) est configuré de manière à faire glisser la broche arrondie hors de l'encliquetage (308) en cas de rotation dans le sens de rotation opposé.

11. Lunettes vidéo (105, 300) selon l'une quelconque des revendications précédentes, dans lesquelles l'encliquetage (308) comprend une encoche.

12. Lunettes vidéo (105, 300) selon la revendication 11, dans lesquelles l'encoche est fixée à la pointe d'un nez.

13. Lunettes vidéo (105, 300) selon l'une quelconque des revendications précédentes, dans lesquelles l'articulation (304) comprend une articulation à parallélogramme.

14. Lunettes vidéo (105, 300) selon l'une quelconque des revendications précédentes, dans lesquelles les lunettes vidéo (105) comprennent un capteur d'orientation pouvant être connecté aux lunettes vidéo (105).

15. Stéréomicroscope (100) comprenant des lunettes vidéo (105, 300) selon l'une quelconque des revendications précédentes.
